# EUROPÄISCHE PATENTSCHRIFT

(11) **EP 1 193 399 B1**
(45) Veröffentlichungstag und Bekanntmachung des Hinweises auf die Patenterteilung: **26.04.2006**
(21) Anmeldenummer: 01116992.7
(22) Anmeldetag: 12.07.2001
(51) Int. Cl.: F04D 29/42, F04D 29/28, F04D 29/66

(54) **Radialgebläse, insbesondere für Beatmungsgeräte**
Radial fan , in particular for respirator
Ventilateur radial, en particulier pour respirateur

(30) Priorität: 28.09.2000 DE 20016769 U
(43) Veröffentlichungstag der Anmeldung: 03.04.2002
(73) Patentinhaber: ebm-papst St. Georgen GmbH & Co. KG, 78112 St. Georgen (DE)
(72) Erfinder: Eimer, Georg, Dipl.-Ing., 78112 St. Georgen (DE); Fehrenbacher, Wolfgang, 78112 St. Georgen (DE); Schmider, Fritz, 78132 Hornberg (DE)

(56) Entgegenhaltungen:
- EP-A- 0 316 470
- EP-A- 0 872 643
- GB-A- 557 312
- GB-A- 629 455
- US-A- 3 289 923
- US-A- 5 797 727

## Beschreibung

Die vorliegende Erfindung betrifft ein Radialgebläse, insbesondere für ein Beatmungsgerät, mit einem Gebläsegehäuse mit einer zentralen, axialen Einlaßöffnung und einer radialen oder etwa tangentialen Auslaßöffnung sowie mit einem motorisch antreibbaren, axial zwischen der Einlaßöffnung und der Auslaßöffnung angeordneten, mit einem zentrischen Einströmbereich der Einlaßöffnung benachbarten Radial-Laufrad, wobei insbesondere der Einlaßöffnung gehäuseaußenseitig eine Ansaugspirale ("Zuluftstromschnecke") derart vorgeordnet ist, dass ein Medium radial oder etwa tangential in einen Spiralkanal einströmt und aus diesem axial in die zentrale Einlaßöffnung übergeht.

Die DE 197 15 581 C1 bzw. die parallele EP 0 872 643 A2, die dem Oberbegriff des Anspruchs 1 entspricht beschreibt, eine Luftzuführung für Gebläse von Geräten zur Beatmung, wie zur Behandlung von Schlafapnoe, wobei unter Zwischenschaltung des Gebläses ein Luftzuführkanal mit einem Luftausgang verbunden ist. Dabei dient die Ansaugspirale zur Reduzierung von durch die Luftströmung hervorgerufenen Geräuschen. Die bekannte Ansaugspirale ist dadurch gekennzeichnet, dass der spiralförmige Luftzuführkanal in mindestens zwei Teilkanäle aufgeteilt ist. Die so entstehenden Teilkanäle enden direkt in der Nähe der zentralen Einlaßöffnung, so dass die einströmende Luft auch direkt, d.h. im Wesentlichen radial in den Einströmbereich des Laufrades gelangt und dort auf die inneren Enden der Laufrad-Schaufeln trifft. Die Luft strömt dann durch das Laufrad radial nach außen und nachfolgend axial in Richtung der Auslaßöffnung.

Die EP 0 316 470 A1 beschreibt ein Radialgebläse für gasförmige Medien mit einem drehbar angeordneten Radial-Laufrad, welches eine hintere Deckscheibe, Radial-Laufschaufeln, eine vordere Deckscheibe, sowie eine Aus- und Einlassöffnung aufzeigt. Dabei enden die Laufschaufeln des Laufrades unmittelbar vor der Innenfläche der Umfangswandung des Gehäuses um somit die Lüftströmung zwischen Ende der Laufschaufeln und Umfangswandung in radialer Richtung so gering wie möglich zu halten. Durch diese erfindungsgemäße Ausgestaltung kann das in radialer Richtung von den Laufschaufeln beschleunigte Medium bereits in axialer Richtung aus dem Laufrad austreten, d.h. es erfolgt praktisch schon "innerhalb" des Laufrades die Umlenkung von der radialen in die axiale Richtung. Nachfolgend kann das Medium über einen eckigen Auslasskanal aus dem Gehäuse ausströmen.

Der vorliegenden Erfindung liegt die Aufgabe zugrunde, bei einem derartigen Radialgebläse die Geräuschemission noch weitergehend zu reduzieren.

Nach der Erfindung wird diese Aufgabe bei einem Gebläse der eingangs genannten Art dadurch gelöst, dass der Spiralkanal von einer entsprechend spiralförmigen Kanalwandung begrenzt ist, die in der Nähe der zentralen Einlassöffnung derart endet, dass jede gedachte an die Kanalwandung im Bereich von deren innerem Ende angelegte Tangente außerhalb der Einlassöffnung oder allenfalls tangential zu dieser verläuft. Dabei wird der Einströmbereich des Laufrades durch die radial inneren Enden von Schaufeln gebildet, wobei diese inneren Enden der Schaufeln auf einem Kreis liegen, dessen Durchmesser mindestens gleich dem, vorzugsweise aber größer als der Durchmesser der Einlassöffnung ist.

Diese erfindungsgemäße Maßnahme beruht auf der Kenntnis, dass durch eine (wegen der forderungsgemäß sehr flachen Bauweise notwendige) seitliche Luftzuführung beim Stand der Technik deshalb hörbare Geräusche verursacht werden, weil der aus der Ansaugspirale in den zentralen Einströmbereich gelangende Luftstrom direkt etwa radial auf die inneren Enden der Radialschaufeln trifft und dadurch deutliche Geräusche verursacht. Es kann davon gesprochen werden, dass der Luftstrom "die inneren Schaufelenden sieht." Gemäß Anspruch 1 ist aber der einzige spiralförmige Ansaugkanal so gestaltet, dass der Luftstrom sich im wesentlichen tangential an die zentrale Öffnung zum Laufrad anlegt, so dass der Luftstrom nicht direkt auf die Radialschaufeln auftrifft, sondern vielmehr mit einem sanften Übergang. Dies bedeutet, dass sich die Luft aus dem spiralförmigen Ansaugkanal kommend praktisch geräuschlos tangential in die zentrale Einlassöffnung und in den Einströmbereich "hineinschleicht."

Eine bevorzugte Weiterbildung der Erfindung ist Gegenstand des Patentanspruchs 2. Hierbei hat das Laufrad auf seiner der Auslassöffnung zugekehrten Axialseite eine Deckscheibe mit einem vorgegebenen Durchmesser in der Weise, dass zwischen dem Außenumfang der Deckscheibe und einer inneren Gehäuse-Umfangsfläche ein verengter Strömungsspalt für das in Richtung Auslassöffnung strömende Medium gebildet wird. Es hat sich gezeigt, dass auf diese Weise eine deutliche Geräuschreduzierung erreicht wird. Durch den verengten Strömungsspalt wird ein ringkammerförmiger Beruhigungsraum gebildet, der das Laufrad umschließt und in dem das aus dem Laufrad strömende Medium zunächst beruhigt wird und dann über den verengten Strömungsspalt in Richtung eines die Auslassöffnung aufweisenden, ebenfalls ringkammerförmigen Ausströmraumes strömt.

Dabei wird der Querschnitt des Strömungsspaltes bevorzugt gemäß Anspruch 3 ausgebildet. Es hat sich gezeigt, dass hierdurch bei einer realisierten Ausführungsform im Vergleich zu einem bekannten Gebläse ein um etwa 13 dBA reduzierter Geräuschpegel erzielt werden konnte, wobei sich durch die Maßnahmen gemäß Anspruch 1 etwa die Hälfte dieser Verbesserung ergibt, und die andere Hälfte auf die Maßnahmen gemäß Anspruch 2 und 3 zurückzuführen sein dürfte. Gerade bei Beatmungsgeräten, die häufig für schlafende Personen verwendet werden (Schlafapnoe) ist ein ruhiger Lauf sehr wichtig, um den Patienten nicht aufzuwecken, wenn das Gebläse anläuft.

Es sei noch erwähnt, dass sich die erfindungsgemäßen Maßnahmen hauptsächlich für Gebläse mit relativ hoher Druckerhöhung und kleinem Volumenstrom eignen, weil sie dafür den besten Schallreduzierungseffekt bringen. Dazu wird auf der Gebläse-Kennlinie ein Arbeitspunkt gewählt, in dem als Nennvolumenstrom ein relativ kleiner Volumenstrom und ein relativ hoher Druck gegeben sind. Der Nennvolumenstrom liegt dann zu einem frei blasenden Volumenstrom in einem Verhältnis von etwa 1 : 12 bis 1 : 6, entsprechend etwa 8 bis 16 %. In einer vorteilhaften Ausführungsform können beispielsweise der Nennvolumenstrom 50 l/min und der frei blasende Volumenstrom 400 l/min betragen, woraus ein Verhältnis von 1 : 8 entsprechend 12, 5 % resultiert.

Weitere vorteilhafte Ausgestaltungsmerkmale der Erfindung sind in den übrigen Unteransprüchen sowie der folgenden Beschreibung enthalten. Anhand eines in der Zeichnung veranschaulichten, bevorzugten Ausführungsbeispiels soll die Erfindung genauer erläutert werden. Dabei zeigen:
- Fig. 1: einen Axialschnitt durch ein erfindungsgemäßes Radialgebläse,
- Fig. 2: eine gesonderte Schnittdarstellung der Ansaugspirale, analog zu Fig. 1,
- Fig. 3: eine Draufsicht auf die Innenseite der Ansaugspirale in Pfeilrichtung III gemäß Fig. 2,
- Fig. 4: eine verkleinerte Schnittdarstellung nur des Radial-Laufrades analog zu Fig. 1 (Schnitt in der Ebene IV gemäß Fig. 5) und
- Fig. 5: eine Draufsicht auf die Einlaßseite des Laufrades in Pfeilrichtung V gemäß Fig. 4.

In den verschiedenen Figuren der Zeichnung sind gleiche Teile stets mit den gleichen Bezugszeichen versehen und brauchen daher in der Regel auch jeweils nur einmal beschrieben zu werden.

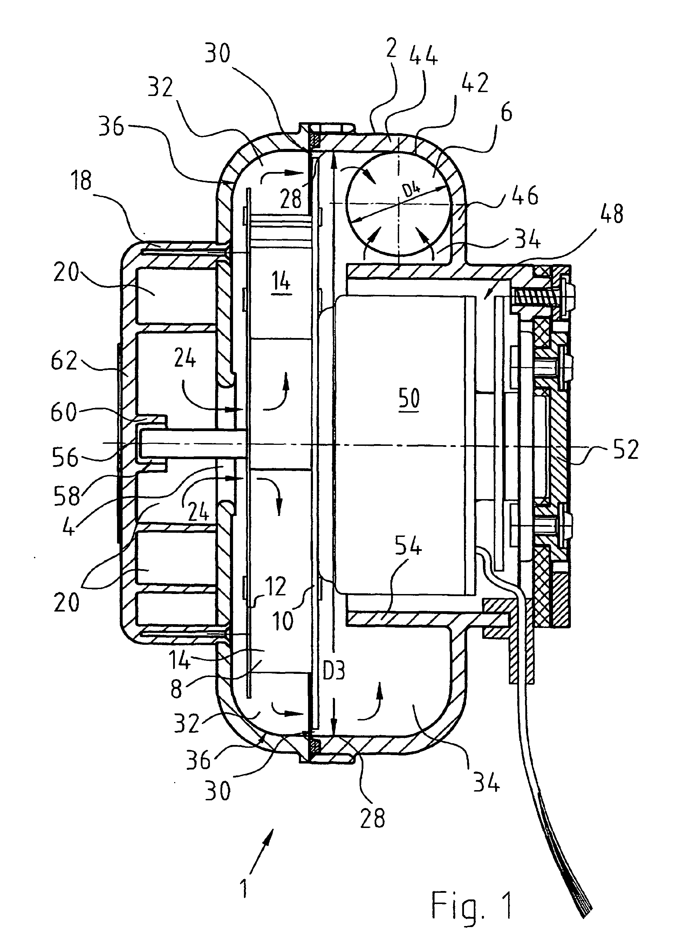

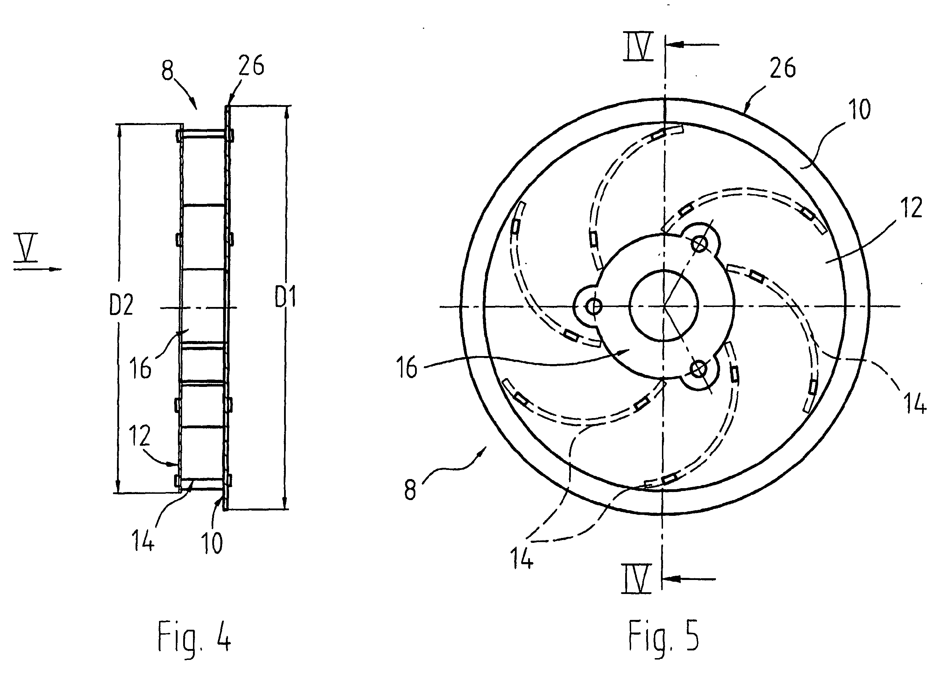

Wie sich aus Fig. 1 ergibt, weist ein erfindungsgemäßes Radialgebläse 1 ein Gebläsegehäuse 2 mit einer zentralen, axialen Einlaßöffnung 4 und einer dazu in axialer Richtung versetzt angeordneten, radialen oder etwa tangentialen Auslaßöffnung 6 auf. Innerhalb des Gebläsegehäuses 2 ist axial zwischen der Einlaßöffnung 4 und der Auslaßöffnung 6 ein Radial-Laufrad 8 angeordnet (vgl. dazu auch Fig. 4 und 5). Das Laufrad 8 besteht aus einer ersten, auslaßseitigen Deckscheibe 10, einer zweiten, einlaßseitigen Deckscheibe 12 sowie zwischen den Deckschreiben 10, 12 angeordneten Radialschaufeln 14. Gemäß Fig. 5 handelt es sich um - je nach Drehrichtung - vorwärts, insbesondere aber rückwärts gekrümmte Schaufeln 14. Die Radiaischaufein 14 definieren mit ihren radial inneren Enden einen zentralen Einströmbereich 16 des Laufrades 8, der direkt benachbart im Bereich der Einlaßöffnung 4 liegt.

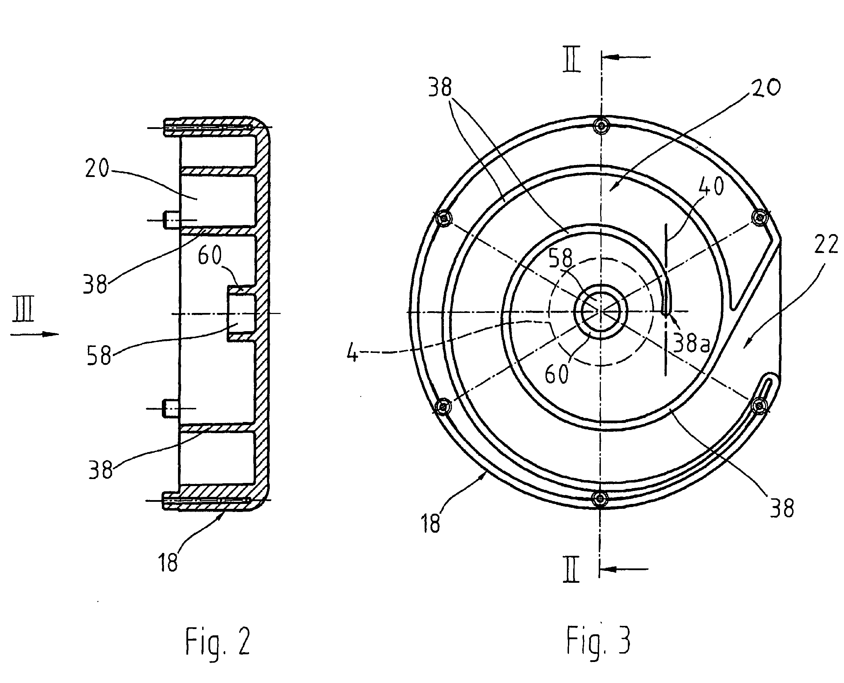

Der Einlaßöffnung 4 ist gehäuseaußenseitig eine Ansaugspirale 18 derart vorgeordnet, dass das jeweilige Medium, insbesondere Luft, radial oder etwa tangential in einen Spiralkanal 20 einströmt (vgl. dazu den Pfeil 22 in Fig. 3) und aus diesem axial in die zentrale Einlaßöffnung 4 übergeht (Pfeile 24 in Fig. 1). Die Ansaugspirale 18 dient in an sich bekannter Weise zur Reduzierung der Ansauggeräusche.

Wie sich weiterhin aus Fig. 1 ergibt, ist erfindungsgemäß die erste, auslaßseitige Deckschreibe 10 mit einem derart großen Durchmesser D1 (siehe Fig. 4) ausgebildet, dass zwischen dem Außenumfang 26 der Deckscheibe 10 und einer inneren Gehäuse-Umfangsfläche 28 ein verengter ringförmiger Strömungsspalt 30 für das zunächst radial über das Laufrad 8 und dann axial in Richtung der Auslaßöffnung 6 strömende Medium gebildet ist. Durch diesen Strömungspalt 30 wird ein das Laufrad 8 umfangsgemäß umschließender Beruhigungsraum 32 gebildet, in dem das radial aus dem Laufrad 8 austretende Medium beruhigt wird und dann über den erfindungsgemäßen Strömungsspalt 30 axial in Richtung eines die Auslaßöffnung 6 aufweisenden Ausströmraumes 34 strömt. Der Strömungsspalt 30 weist einen ringförmigen Strömungsquerschnitt A_{R} auf, der etwa gleich oder sogar etwas kleiner bis maximal etwa 60 % größer als der Strömungsquerschnitt A_{A} der Auslaßöffnung 6 ist. Es gilt somit: A_{R} ≤ A_{A} bis A_{R} = ca. 1,6 x A_{A}.

Der Durchmesser D1 der ersten, den verengten Strömungsspalt 30 bildenden Deckscheibe 10 ist bevorzugt größer als der Durchmesser D2 der zweiten, einlaßseitigen Deckscheibe 12 (siehe Fig. 4). Dadurch kann das Gehäuse 2 vorteilhafterweise in seinem das Laufrad 8 umfangsgemäß umschließenden Bereich eine - im Axialschnitt gesehen; siehe Fig. 1 - konkav gekrümmte Innenfläche 36 derart aufweisen, dass die radial aus dem Laufrad 8 kommende Luft von der konkaven Innenfläche 36 strömungsgünstig in Richtung des verengten Strömungsspaltes 30 geleitet wird.

In einer konkret realisierten Ausführungsform des erfindungsgemäßen Radialgebläses sind beispielsweise folgende Abmessungen gegeben:

| | |
|---|---|
| Außendurchmesser D1 der ersten Deckscheibe 10: | 100,6 mm |
| Außendurchmesser D2 der zweiten Deckscheibe 12: Innendurchmesser D3 des Gehäuses im Bereich des | 90,0 mm |
| Strömungsspaltes 30 bzw. der inneren Umfangsfläche 28: | 103,0 mm |
| Innendurchmesser D4 der Auslaßöffnung 6 (Fig. 1): | 18,0 mm |

Dabei liegen die Radialschaufeln 14 mit ihren äußeren Enden auf einem Kreis mit einem Durchmesser von 89,0 mm.

Aus diesen lediglich beispielhaft genannten Abmessungen ergibt sich ein Verhältnis der Querschnittsflächen Auslaßöffnung 6 zu Strömungsspalt 30 von etwa 2 : 3. Somit ist der Strömungsquerschnitt A_{R} des Strömungsspaltes 30 etwa 50 % größer als der Strömungsquerschnitt A_{A} der Auslaßöffnung 6, es gilt A_{R} ≈ 1,5 · A_{A}.

Gemäß Fig. 1 wird dabei die Auslaßöffnung 6 von einer axialen Projektion der ersten Deckscheibe 10 radial nahezu vollständig überdeckt. Die durch den Strömungsspalt 30 strömende Luft verteilt sich deshalb zunächst unter Beruhigung innerhalb des Ausströmraumes 34 und strömt dann in Richtung der in Fig. 1 eingezeichneten Pfeile in die Auslaßöffnung 6.

Anhand der Fig. 3 soll nun ein weiterer erfindungsgemäßer Aspekt beschrieben werden. Demnach wird der Spiralkanal 20 von einer entsprechend spiralförmigen Kanalwandung 38 begrenzt, die in der Nähe der zentralen Einlaßöffnung 4 (in Fig. 3 gestrichelt eingezeichnet) derart endet, dass jede gedachte an die Kanalwandung 38 - und zwar insbesondere auch an deren Ende 38a - angelegte Tangente (vgl. beispielsweise die Tangente 40 in Fig. 3) außerhalb der Einlaßöffnung 4 und des Einströmbereichs 16 des Laufrades 8 oder allenfalls tangential dazu liegt. Durch diese erfindungsgemäße Ausgestaltung wird vermieden, dass die aus dem Spiralkanal 20 in die Einlaßöffnung 4 übergehende Luft direkt auf die inneren Enden der Radialschaufeln 14 auftrifft. Somit werden vorteilhafterweise auch entsprechende Geräusche vermieden, indem sich die aus der Ansaugspirale 18 kommende Luft praktisch geräuschlos in die zentrische Einlaßöffnung 4 und in den Einströmbereich 16 des Laufrades 8 "hineinschleicht". Es wird somit eine im Bereich der Einlaßöffnung 4 rotierende, strudelartige Luftströmung erzeugt, was zum Übergang in die radiale Strömung durch das Laufrad 8 besonders günstig ist. Dabei ist es zudem vorteilhaft, wenn die Rotationsrichtung des Laufrades 8 mit der durch den Spiralkanal 20 erzeugten Drallrichtung übereinstimmt. Dabei liegen die den Einströmbereich 16 bildenden, radial inneren Enden der Radialschaufeln 14 auf einem Kreis, dessen Durchmesser mindestens gleich dem, vorzugsweise aber etwas größer als der Durchmesser der Einlaßöffnung 4 ist. Es wird ein strömungsgünstiger und daher geräuscharmer Übergang von der Ansaugspirale 18 zum Laufrad 8 erreicht.

Die äußeren Enden der Radialschaufeln 14 des Laufrades 8 liegen auf einem Kreis, dessen Durchmesser etwa dem Durchmesser D2 der einlaßseitigen, zweiten Deckscheibe 12 entspricht. Somit lassen die Radialschaufeln 14 vorteilhafterweise den oben erläuterten Beruhigungsraum 32 frei.

Wie sich noch aus Fig. 1 ergibt, grenzt die Auslaßöffnung 6 bevorzugt direkt an eine innere, ähnlich der Innenfläche 36 konkav gekrümmte Gehäusefläche 42 im Übergang zwischen einer Umfangswandung 44 und einer radialen Stirnwandung 46 des Gehäuses an. Auch dies ist sehr strömungsgünstig.

Weiterhin ergibt sich aus Fig. 1, dass das Gebläsegehäuse 2 auf seiner der Einlaßöffnung 4 und der Ansaugspirale 18 gegenüber liegenden Seite einen Aufnahmeraum 48 für einen Elektromotor 50 bildet. Der Elektromotor 50 wird innerhalb des Aufnahmeraums 48 von einem Motorflansch 52 gehalten, der gleichzeitig als Verschlußdeckel fungiert. Zudem wird der Aufnahmeraum 48 auch innerhalb des Gebläsegehäuses 2 durch eine Ringwandung 54 begrenzt, die sich axial in Richtung des Laufrades 8 erstreckt und somit ringförmig bzw. umfangsgemäß von dem Ausströmraum 34 umschlossen wird. Dabei ist es weiterhin vorteilhaft, wenn der Rotor einen über das Laufrad 8 axial hinausragenden Wellenabschnitt 56 aufweist, der mit seinem freien Ende in einer Lageraufnahme 58 geführt ist. Vorzugsweise ist die Lageraufnahme 58 Bestandteil der Ansaugspirale 18, indem sie beispielsweise durch einen einstückigen ringförmigen Ansatz 60 einer radialen Spiralgehäuse-Stirnwandung 62 gebildet ist. Durch diese vorteilhafte Maßnahme wird das Laufrad 8 gegen Stoßbelastungen geführt, was insbesondere wegen des relativ engen Strömungsspaltes 30 von Bedeutung ist, um auch bei Stoßbelastungen Schleifgeräusche des Laufrades 8 bzw. der Deckscheibe 10 an der inneren Umfangsfläche 28 zu vermeiden. Dazu ist das Führungsspiel des Wellenabschnittes 56 innerhalb der Lageraufnahme 58 jedenfalls kleiner als der Strömungsspalt 30.

Mit den beschriebenen erfindungsgemäßen Maßnahmen wird eine deutliche Reduzierung der Strömungsgeräusche erreicht. Dies gilt besonders auch im Falle eines Rückluftstromes, beispielsweise wenn bei der Anwendung für ein Beatmungsgerät durch ein Ausatmen der Luftstrom zurück kommt. Durch die Erfindung wird besonders auch ein solcher Rückluftstrom beruhigt, so dass die Geräusche wesentlich vermindert werden. Dabei sind die erfindungsgemäßen Maßnahmen vor allem bei solchen Gebläsen geeignet, bei denen der Nennvolumenstrom zu einem freiblasenden Volumenstrom in einem Verhältnis von etwa 1 : 12 bis 1 : 6, entsprechend etwa 8 : 16 %, liegt.

## Patentansprüche

1. Radialgebläse (1), insbesondere für ein Beatmungsgerät, mit einem Gebläsegehäuse (2) mit einer zentralen, axialen Einlassöffnung (4) und einem innerhalb des Gebläsegehäuses (2) mit einem zentrischen Einströmbereich (16) der Einlassöffnung (4) benachbarten Radial-Laufrad (8), wobei der Einlassöffnung (4) gehäuse außenseitig eine Ansaugspirale (18) derart vorgeordnet ist, dass ein Medium radial oder etwa tangential in einen Spiralkanal (20) der Ansaugspirale (18) einströmt und aus diesem axial in die zentrale Einlassöffnung (4) übergeht,
**dadurch gekennzeichnet, dass** der Einströmbereich (16) des Laufrades (8) durch die radial inneren Enden von Schaufeln (14) des Radial-Laufrades (8) gebildet wird, wobei diese inneren Enden der Schaufeln (14) auf einem Kreis liegen, dessen Durchmesser mindestens gleich dem, vorzugsweise aber größer als der Durchmesser der Einlassöffnung (4) ist,
und dass die Ansaugspirale (18) einen einzigen spiralförmigen Ansaugkanal (20) aufweist, welcher durch eine spiralförmige Kanalwandung (38) begrenzt ist, die in der Nähe der zentralen Einlassöffnung (4) derart endet, dass jede gedachte an diese Kanalwandung (38) im Bereich von deren inneren Ende (38a) angelegte Tangente (40) außerhalb der Einlassöffnung (4) und des Einströmbereichs (16) oder allenfalls tangential zu dieser verläuft.

2. Radialgebläse nach Anspruch 1
**dadurch gekennzeichnet, dass** das Radial-Laufrad (8) axial zwischen der zentralen Einlassöffnung (4) und einer kreisförmigen oder etwa tangentialen Auslassöffnung (6) des Gebläsegehäuses (2) angeordnet ist, wobei das Radial-Laufrad (8) auf seiner der Auslassöffnung (6) zugekehrten Axialseite eine Deckscheibe (10) mit einem vorgegebenen Durchmesser (D1) aufweist, so dass zwischen dem Außenumfang (26) der Deckscheibe (10) und einer inneren Gehäuse-Umfangsfläche (28) ein verengter Strömungsspalt (30) für das vom Radial-Laufrad (8) in Richtung der Auslassöffnung (6) strömende Medium gebildet wird.

3. Radialgebläse nach Anspruch 2,
**dadurch gekennzeichnet, dass** der Strömungsspalt (30) einen ringförmigen Strömungsquerschnitt (A_{R}) aufweist, der kleiner bis maximal 60 % größer als der Strömungsquerschnitt (A_{A}) der Auslassöffnung (6), ist.

4. Radialgebläse nach einem der Ansprüche 1 bis 3,
**dadurch gekennzeichnet, dass** das Gehäuse (2) in seinem dem Laufrad (8) umfangsgemäß umschließenden Bereich eine konkav gekrümmte Innenfläche (36) aufweist, so dass das radial aus dem Laufrad (8) kommende Medium von der konkaven Innenfläche (36) strömungsgünstig in Richtung des Strömungsspaltes (30) geleitet wird.

5. Radialgebläse nach einem der Ansprüche 1 bis 4,
**dadurch gekennzeichnet, dass** das Laufrad (8) mit einem Rotor eines Elektromotors (50) verbunden ist, wobei der Elektromotor (50) in das Gebläsegehäuse (2) integriert ist.

6. Radialgebläse nach Anspruch 5,
**dadurch gekennzeichnet, dass** der Rotor einen über das Laufrad (8) hinausragenden Wellenabschnitt (56) aufweist, der mit seinem freien Ende in einer Lageraufnahme (58) geführt ist, wobei die Lageraufnahme (58) vorzugsweise Bestandteil der Ansaugspirale (18) ist.

7. Radialgebläse nach einem der Ansprüche 1 bis 6,
**dadurch gekennzeichnet, dass** das Laufrad (8) Radialschaufeln (14) aufweist, deren radial äußere Enden auf einem Kreis liegen, dessen Durchmesser etwa dem Durchmesser (D2) der einlassseitigen Deckscheibe (12) des Radial Laufrades (8) entspricht.

8. Radialgebläse nach einem der Ansprüche 1 bis 7,
**dadurch gekennzeichnet, dass** die Auslassöffnung (6) direkt an eine innere, konkav gekrümmte Gehäusefläche (42) im Übergang zwischen einer Umfangswandung (44) und einer radialen Stirnwandung (46) angrenzt.

9. Radialgebläse nach einem der Ansprüche 1 bis 8,
**dadurch gekennzeichnet, dass** ein Gebläse - Arbeitspunkt in einem Bereich der Gebläse - Kennlinie liegt, in dem als Nennvolumenstrom ein relativ kleiner Volumenstrom und ein relativ hoher Druck gegeben ist.

10. Radialgebläse nach einem der Ansprüche 1 bis 9,
**dadurch gekennzeichnet, dass** der Nennvolumenstrom zu einem freiblasenden Volumenstrom in einem Verhältnis von etwa 1:12 bis 1:6, entsprechend etwa 8 bis 16 % liegt.

## Claims

1. Radial fan (1), in particular for a respirator, with a fan housing (2) with a central axial inlet opening (4) and inside the fan housing (2) a radial wheel (8) with a central inflow area (16) neighbouring the inlet opening (4), wherein an intake helix (18) is arranged on the outside of the housing in front of the inlet opening (4) such that a medium flows radially or roughly tangentially into a helical channel (20) of the intake helix (18) and passes axially out of this into the central inlet opening (4),
**characterised in that** the inflow area (16) of the wheel (8) is formed by the radially inner ends of blades (14) of the radial wheel (8), these inner ends of the blades (14) lying on a circle the diameter of which is at least equal to but preferably greater than the diameter of the inlet opening (4),
and **in that** the intake helix (18) comprises a single helical intake channel (20) which is bounded by a helical channel wall (38) which ends in the vicinity of the central inlet opening (4) such that any imaginary tangent (40) located on this channel wall (38) in the area of its inner end (38a) runs outside the inlet opening (4) and the inflow area (16) or at least tangentially to this.

2. Radial fan according to claim 1,
**characterised in that** the radial wheel (8) is arranged axially between the central inlet opening (4) and a circular or roughly tangential outlet opening (6) of the fan housing (2), wherein the radial wheel (8) comprises a covering disc (10) with a predetermined diameter (D1) on its axial side facing the outlet opening (6) so that a narrowed flow gap (30) is formed between the outside circumference (26) of the covering disc (10) and an inner housing circumferential face (28) for the medium flowing from the radial wheel (8) in the direction of the outlet opening (6).

3. Radial fan according to claim 2,
**characterised in that** the flow gap (30) has an annular flow cross section (A_{R}) which is smaller than up to a maximum of 60% greater than the flow cross-section (A_{A}) of the outlet opening (6).

4. Radial fan according to one of claims 1 to 3,
**characterised in that** in its area circumferentially surrounding the wheel (8) the housing (2) comprises a concavely curved internal face (36) so that the medium coming radially out of the wheel (8) is ducted in the direction of the flow gap (30) by the concave internal face (36) in a manner favourable for flow.

5. Radial fan according to one of claims 1 to 4,
**characterised in that** the wheel (8) is connected to a rotor of an electric motor (50), the electric motor (50) being incorporated in the fan housing (2).

6. Radial fan according to claim 5,
**characterised in that** the rotor comprises a shaft portion (56) which projects beyond the wheel (8) and is guided by its free end in a bearing housing (58), the bearing housing (58) preferably being part of the intake helix (18).

7. Radial fan according to one of claims 1 to 6,
**characterised in that** the wheel (8) comprises radial blades (14) the radially outer ends of which lie on a circle the diameter of which roughly corresponds to the diameter (D2) of the covering disc (12) on the inlet side of the radial wheel (8).

8. Radial fan according to one of claims 1 to 7,
**characterised in that** the outlet opening (6) directly adjoins an inner, concavely curved housing face (42) in the transition between a circumferential wall (44) and a radial end wall (46).

9. Radial fan according to one of claims 1 to 8,
**characterised in that** a fan working point lies in an area of the fan characteristic in which the nominal volume flow is a relatively small volume flow with a relatively high pressure.

10. Radial fan according to one of claims 1 to 9,
**characterised in that** the nominal volume flow lies in a ratio of approximately 1:12 to 1:6, corresponding to roughly 8 to 16%, in relation to a free-blowing volume flow.

## Revendications

1. Ventilateur radial (1), en particulier pour un respirateur, avec un boîtier de ventilateur (2) présentant une ouverture d'entrée axiale centrale (4) et une roue radiale (8) voisine d'une zone d'admission centrale (16) de l'ouverture d'entrée (4) à l'intérieur du boîtier de ventilateur (2), dans lequel une spirale d'aspiration (18) précède l'ouverture d'entrée (4) du côté extérieur du boîtier de telle manière qu'un fluide afflue radialement ou à peu près tangentiellement dans un canal en spirale (20) de la spirale d'aspiration (18) et sort de celui-ci axialement pour passer dans l'ouverture d'entrée centrale (4),
**caractérisé par le fait que** la zone d'admission (16) de la roue (8) est formée par les extrémités radialement intérieures des ailettes (14) de la roue radiale (8), ces extrémités intérieures des ailettes (14) étant situées sur un cercle dont le diamètre est au moins égal, mais de préférence supérieur au diamètre de l'ouverture d'entrée (4),
et que la spirale d'aspiration (18) présente un seul canal d'aspiration en spirale (20) qui est limité par une paroi de canal en spirale (38) qui se termine à proximité de l'ouverture d'entrée centrale (4) de telle manière que toute tangente (40) imaginaire à cette paroi de canal (38) tracée au niveau de son extrémité intérieure (38a) passe à l'extérieur de l'ouverture d'entrée (4) et de la zone d'admission (16) ou tout au plus tangentiellement à celles-ci.

2. Ventilateur radial selon la revendication 1,
**caractérisé par le fait que** la roue radiale (8) est disposée axialement entre l'ouverture d'entrée centrale (4) et une ouverture de sortie (6) circulaire ou à peu près tangentielle du boîtier de ventilateur (2), la roue radiale (8) présentant sur son côté axial tourné vers l'ouverture de sortie (6) un disque de recouvrement (10) d'un diamètre (D1) défini, de manière à former entre la circonférence extérieure (26) du disque de recouvrement (10) et une surface circonférentielle intérieure (28) du boîtier une fente d'écoulement (30) rétrécie pour le fluide qui s'écoule de la roue radiale (8) en direction de l'ouverture de sortie (6).

3. Ventilateur radial selon la revendication 2,
**caractérisé par le fait que** la fente d'écoulement (30) présente une section d'écoulement annulaire (A_{R}) qui est plus petite ou jusqu'à 60 % au maximum plus grande que la section d'écoulement (A_{A}) de l'ouverture de sortie (6).

4. Ventilateur radial selon l'une des revendications 1 à 3,
**caractérisé par le fait que** le boîtier (2) présente dans sa partie entourant circonférentiellement la roue (8) une surface intérieure (36) incurvée concave, de sorte que le fluide venant radialement de la roue (8) est canalisé de manière aérodynamique en direction de la fente d'écoulement (30) par la surface intérieure concave (36).

5. Ventilateur radial selon l'une des revendications 1 à 4,
**caractérisé par le fait que** la roue (8) est reliée à un rotor d'un moteur électrique (50), lequel moteur électrique (50) est intégré dans le boîtier de ventilateur (2).

6. Ventilateur radial selon la revendication 5,
**caractérisé par le fait que** le rotor présente une section d'arbre (56) dépassant de la roue (8) qui est guidée par son extrémité libre dans un logement de palier (58), lequel logement de palier (58) fait de préférence partie de la spirale d'aspiration (18).

7. Ventilateur radial selon l'une des revendications 1 à 6,
**caractérisé par le fait que** la roue (8) présente des ailettes radiales (14) dont les extrémités radialement extérieures sont situées sur un cercle dont le diamètre correspond à peu près au diamètre (D2) du disque de recouvrement (12) côté entrée de la roue radiale (8).

8. Ventilateur radial selon l'une des revendications 1 à 7,
**caractérisé par le fait que** l'ouverture de sortie (6) est directement adjacente à une surface de boîtier (42) intérieure, incurvée concave, à la jonction entre une paroi circonférentielle (44) et une paroi frontale radiale (46).

9. Ventilateur radial selon l'une des revendications 1 à 8,
**caractérisé par le fait qu'**un point de fonctionnement du ventilateur se situe dans une partie de la courbe caractéristique du ventilateur dans laquelle il existe comme débit volumique nominal un débit volumique relativement petit et une pression relativement élevée.

10. Ventilateur radial selon l'une des revendications 1 à 9,
**caractérisé par le fait que** le débit volumique nominal est dans un rapport d'environ 1:12 à 1 :16, correspondant à environ 8 à 16 %, avec un débit volumique en soufflage libre.
